# EUROPÄISCHE PATENTANMELDUNG

(11) **EP 3 569 288 A1**
(43) Veröffentlichungstag der Anmeldung: **20.11.2019**
(21) Anmeldenummer: 19173784.0
(22) Anmeldetag: 10.05.2019
(51) Int. Cl.: A61N 5/06

(54) **LICHTWELLEN-THERAPIEVORRICHTUNG**

(30) Priorität: 10.05.2018 DE 202018002388 U
(71) Anmelder: Steins, Markus, 33106 Paderborn (DE)
(72) Erfinder: Steins, Markus, 33106 Paderborn (DE)
(74) Vertreter: Meinke, Jochen

(57) **Zusammenfassung**

Die Erfindung betrifft eine Lichtwellen-Therapievorrichtung, welche durch einen auf die Haut eines menschlichen oder tierischen Lebewesens sowie auf die Oberfläche eines pflanzlichen Wesens aufbringbaren Therapiekörper (1) ausgebildet ist, aufweisend: eine obere Außenhülle (2), die relativ in Axialrichtung zu einer unteren Außenhülle (3) angeordnet ist, wobei innerhalb der Außenhülle unterschiedliche Arten und eine variable Anzahl von Polarisationsfiltern (4), Farbfiltern (5), Spiegelfläche (6), Ronden (7) und einem Label (8) derart angeordnet sind, dass der Therapiekörper (1) mit den unter der Haut angeordneten Zellen (11) und/oder Organen (11) und/oder Muskeln (9) und/oder Gelenken (10) und/oder anderen Bestandteilen (11) des menschlichen sowie tierischen Körpers oder der Pflanze in Wirkverbindung tritt.

## Beschreibung

Die Erfindung betrifft eine Lichtwellen-Therapievorrichtung, welche durch einen auf die Haut eines menschlichen oder tierischen Lebewesens sowie auf die Oberfläche eines pflanzlichen Wesens aufbringbaren Therapiekörper ausgebildet ist. Der Therapiekörper weist in seinem Aufbau verschiedene lichtdurchlässige Filter, verschiedene, je nach Ausführungsform ausgebildete Spiegel(flächen), sowie Polarisationsfilter und Ronden auf. Durch seinen charakteristischen -je nach Ausführungsform- ausgebildeten Aufbau tritt der Therapiekörper mit den unter der Haut angeordneten Zellen und/oder Organen und/oder Muskeln und/oder Gelenken und/oder andern Inhaltsstoffen des menschlichen sowie tierischen Körpers oder der Pflanze in Wirkverbindung.

Aus DE 20 2014 101 021 U1 ist eine Therapievorrichtung bekannt, die aus einem auf die Haut eines Lebewesens auflegbaren Therapiekörper besteht, welche aus einem lichtdurchlässigen Filter und eine Reflexionsfläche zur Wirkverbindung mit unter der Haut angeordneten Zellen und/oder Organen besteht. Hierbei soll die der auflegbare Therapiekörper den Wirkungsbereich auf einfache vergrößern. Nachteilig an der bekannten Therapievorrichtung ist, dass zur Vergrößerung des Wirkungsbereiches, das heißt um eine optimale Wirkung der Therapie zu erreichen immer zwei Therapievorrichtungen in einem zueinander angebrachten Abstand a notwendig sind. Dies stellt zum einen eine unnötig kompliziert umgesetzte Therapiemaßnahme dar und ist zum anderen mit höheren Anschaffungs- und Behandlungskosten für den/die Patienten verbunden.

Aus der DE 20 2008 011 806 U1 ist eine weitere Therapievorrichtung bekannt, die aus einem plattenförmigen Körper besteht, der auf die Haut eine Lebewesens auflegbar ist. Der Therapiekörper weist eine lichtdurchlässige Linse, einen lichtdurchlässigen Filter sowie eine Reflexionsfläche auf, sodass von unter der Haut angeordneten Zellen abgestrahlte Biophotonen, die von dem Therapiekörper erfasst werden, reflektiert werden Biophotonen sind Photonen bzw. Lichtquanten, die von einer Zelle abgestrahlt werden. Eine solche Biophotonenstrahlung bzw. Zellstrahlung ist erstmalig empirisch im Jahre 1922 von dem russischen Mediziner Prof. Alexander Gurwitsch nachgewiesen worden. Die bekannte Vorrichtung geht davon aus, dass die Biophotonen mittels der Reflexionsfläche zurück in Richtung der abstrahlenden Zelle reflektiert werden, wobei die Linse für eine Bündelung der Biophotonen sorgt. Durch die Reflexion der Biophotonen ändert sich der Zustand der Zelle, sodass bei Kommunikation der Zelle mit anderen Zellen die durch die Biophotonen übertragenen Informationen auf den Ursprung zurückgesetzt sind. Nachteilig an der bekannten Therapievorrichtung ist, dass diese punktuell nur auf Zellen wirkt, die im Bereich des Therapiekörpers unterhalb der Haut angeordnet sind.

Aufgabe der vorliegenden Erfindung ist es daher, eine Therapievorrichtung derart weiterzubilden, dass ein auf die Haut eines menschlichen sowie tierischen Lebewesens oder auf die Oberfläche einer Pflanze auflegbarer Therapiekörper entsteht, der eine derart ausgebildete Kombination von Filtern, Spiegelflächen und anderen Bauteilen darstellt, dass durch bereits einen Therapiekörper eine Wirkverbindung mit den unter der Haut angeordneten Zellen und/oder Organen und/ oder Muskeln und/oder Gelenken und/oder andern Inhaltsstoffen des menschlichen sowie tierischen Körpers oder der Pflanze entsteht.

Diese Aufgabe wird mit einer Lichtwellen-Therapievorrichtung mit den Merkmalen des Patentanspruches 1 gelöst.

Der besondere Vorteil der Erfindung besteht darin, dass durch eine Kombination der Bestandteile (Filter, Spiegel und Polarisationsfilter) in einem Therapiekörper eine verbesserte Wirkverbindung mit den unter der Haut angeordneten Zellen und/oder Organen und/oder Muskeln und/oder Gelenken und/oder andern Inhaltsstoffen des menschlichen sowie tierischen Körpers oder der Pflanze auftritt. Erst die Polarisation ermöglicht eine kohärente Rückführung des Lichtes, die entscheidend für eine Wirkung ist. Dadurch, dass ein Therapiekörper alle notwendigen Bestandteile in einem vereint, lassen sich der Therapieaufwand sowie die Therapiekosten deutlich verringern. Es hat sich gezeigt, dass somit bereits ein Therapiekörper ausreicht um eine Behandlung des menschlichen sowie tierischen Körpers oder der Pflanze ausreicht. Dadurch wird den Patienten (menschliches Lebewesen) eine vereinfachte, selbst ausführbare Behandlungsmöglichkeit geboten.

Weitere Vorteile der Erfindung ergeben sich aus den weiteren Unteransprüchen.

Nach einer bevorzugten Ausführungsform der Erfindung besteht der Therapiekörper aus einem Polarisationsfilter, keinem, einem oder einer beliebigen Anzahl an Farbfiltern und einem Spiegel. Der Spiegel ist vorzugsweise als Spiegel mit Polarisationsfilter ausgebildet. Es erfolgt somit eine Polarisation der Lichtstrahlen, sowie eine Selektion des Wellenlängenbereiches, durch Filterung der Lichtstrahlen in unterschiedlichen Frequenzbereichen, sowie eine Reflexion der Lichtstrahlen zu, unter den Auflagestellen angeordneten Zellen des Körpers oder der Pflanze. Vorteilhaft können so, die von den entsprechenden Zellen ausgehenden biochemischen Prozesse und/oder die Kommunikation der Zellen untereinander und/ oder die inneren Abläufe der Zelle und/oder die Unterstützung bzw. Steuerung der Zelle derart günstig verändert werden, dass eine Schmerzlinderung und/oder Schmerzbefreiung und/oder eine Dysfunktion aufgehoben wird und/oder eine Mobilisierung der Bewegungsabläufe eintritt.

Ausführungsbeispiele der Erfindung werden nachfolgend anhand der Zeichnungen näher erläutert. Diese zeigen in
- Fig. 1: eine Gesamtansicht, einen Querschnitt durch den Therapiekörper,
- Fig. 2: eine Gesamtansicht, einen Querschnitt durch den Therapiekörper bezugnehmend auf die dimensionalen Abmessungen,
- Fig. 3: einen Querschnitt durch eine beliebige Therapieanordnung, zur Therapie eines Muskels,
- Fig. 4: einen Querschnitt durch eine beliebige Therapieanordnung, zur Therapie eines Gelenks bzw. Knochens sowie
- Fig. 5: einen Querschnitt durch eine beliebige Therapieanordnung, zur Therapie eines Organs, einer Zelle oder sonstiger Bestandteile des menschlichen oder Tierischen Körpers sowie einer Pflanze.

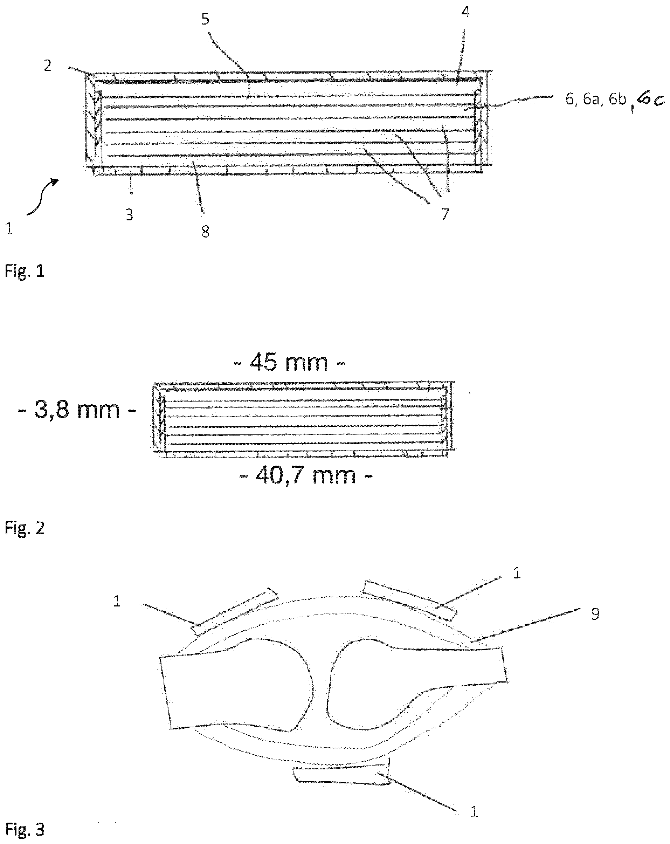

In Fig. 1 sind alle Bestandteile des Therapiekörpers 1 in Gesamtansicht mittels eines Querschnitts aufgezeigt, so wie in Fig. 2 alle äußeren Abmessungen des Therapiekörpers 1 in Gesamtansicht mittels eines Querschnitts aufgezeigt sind.

Eine erfindungsgemäße Lichtwellen-Therapievorrichtung besteht aus einem kreisförmig ausgebildeten Therapiekörper 1, der hohlzylinderförmig ausgebildet ist und einen vorzugsweisen Durchmesser von 45mm und eine vorzugsweise Dicke von 3,8mm aufweist. Der Therapiekörper 1 besteht aus einer oberen Außenhülle 2, welche relativ in Axialrichtung zu einer unteren Außenhülle 3 angeordnet ist.

Die obere Außenhülle 2 ist vorzugsweise stoffschlüssig durch eine klebetechnische Verbindung, vorzugsweise mit einem Klebstoff mit der unteren Außenhülle 3 verbunden. Die obere Außenhülle 2 hat einen vorzugsweisen Durchmesser von 45mm, wobei die untere Außenhülle 3 einen vorzugsweisen Durchmesser von 40,7mm aufweist. Die untere Außenhülle 3 dient als Auflagefläche auf die Haut eines tierischen und/oder menschlichen Lebewesens oder auf die Oberfläche einer Pflanze.

Die Innenseite der oberen Außenhülle 2, dient als Anlagefläche für keinen oder einen Polarisationsfilter 4. Der Polarisationsfilter 4 dient als erster Filter, der die auftreffenden inkohärenten Lichtstrahlen polarisiert und diese in Form von kohärenten Lichtstrahlen an den nächsten Filter, vorzugsweise Farbfilter 5 weiterleitet.

Die Unterseite des Polarisationsfilters 4 dient als Anlagefläche für den Farbfilter 5. Der Farbfilter 5 dient als zweiter Filter, welcher die kohärenten Lichtstrahlen in unterschiedlichen Spektralbereichen an den Spiegel 6 weiterleitet. Es kann kein Farbfilter 5, ein Farbfilter 5 oder mehrere Farbfilter 5 in der Therapievorrichtung verbaut werden. Dadurch können unterschiedliche Spektralbereiche an den Spiegel 6 weiter gegeben werden. Jeder Filter hat aufgrund der verschiedenen Wellenlänge einen anderen Einfluss auf die Zellen. Folgende Farbfilter 5 mit folgenden Wellenlängen können einzeln oder in beliebiger Kombination miteinander eingesetzt werden.

Farbfilter 5 können mit der Farbe Lila den Wellenlängenbereich 380nm bis 420nm, Blau den Wellenbereich 420 nm bis 490nm, Grün den Wellenbereich 490nm bis 575nm, Gelb den Wellenlängenbereich 575nm bis 585nm, Orange den Wellenlängenbereich 585nm bis 650nm, Rot den Wellenlängenbereich 650nm bis 750nm filtern. Weiterhin sind noch Braun als Mischfarbe, Schwarz und Weiß möglich. Ebenso können UV-Lichtstrahlen mit einer Wellenlänge von 10nm bis 380nm und Infrarot-Lichtstrahlen mit einer Wellenlänge von 780nm bis 1mm mithilfe der Farbfilter 5 gefiltert werden. Bei einer Kombination der Farbfilter 5 ergibt sich ebenfalls eine Überlagerung der Filterbereiche der Wellenlängen.

Nachdem die Lichtstrahlen durch den Farbfilter 5 in einem bestimmten Wellenlängenbereich gefiltert wurden, treffen diese auf einen an der Unterseite des Farbfilters 5 anliegenden Spiegel 6. Der Spiegel 6 kann als Oberflächenspiegel 6a oder bevorzugter Weise als Unterflächenspiegel 6b ausgebildet sein, ebenso ist eine Reflexfolie 6c möglich. Durch Auftreffen des kohärenten, sowie Wellenlängen spezifisch gefilterten Lichtes auf den Spiegel 6 werden die bereits doppelt gefilterten Lichtstrahlen zurück in die Zelle geleitet bzw. geworfen.

Bei einem kranken bzw. unter Schmerzen leidenden Patienten (menschliches oder tierisches Lebewesen) befinden sich die Photonen der Zelle in Inkohärenz, wodurch Schmerzen und/oder Dysfunktionen und/oder Bewegungseinschränkungen ausgelöst werden. Durch Zurückleiten des doppelt gefilterten Lichtes durch den Spiegel 6 zurück an die Zelle wird kohärentes Licht in die inkohärenten Zellen geleitet. Dadurch wird die Zelle wieder kohärent und die Schmerzen und/oder Dysfunktionen und/oder Bewegungseinschränkungen werden aufgehoben bzw. gelöst bzw. verbessert.

Auf der Unterseite des Spiegels 6 befinden sich die Ronden 7. Diese sind frei von einer therapeutischen Wirkung. Durch die Ronden 7 wird der Platz zwischen Spiegel 6 und Label 8 beabstandet. Je nach Einsatz des Oberflächenspiegels 6a oder des Unterflächenspiegels 6b, müssen bei Verwendung des Oberflächenspiegels 6a drei Ronden 7 als Platzhalter und bei Verwendung des Unterflächenspiegels 6b eine Ronde 7 als Platzhalter eingesetzt werden. Es ist möglich bis zu zehn Ronden als Platzhalter einzusetzen.

Das Label 8 enthält keine therapeutische Wirkung, sondern dient der äußeren Kenntlichmachung des Therapievorrichtung 1.

In Fig. 3 wird ein Querschnitt durch eine beliebige Therapieanordnung, zur Therapie eines Muskels 9 dargestellt. Die Therapievorrichtung wird dabei auf bestimmte, für die Behandlung des Muskels 9 relevante Akupunkturpunkte, Meridiansysteme, Triggerpunkte, Reflexzonen oder sonstige Punkte auf die Haut des Patienten (menschliches oder tierisches Lebewesen) aufgelegt.

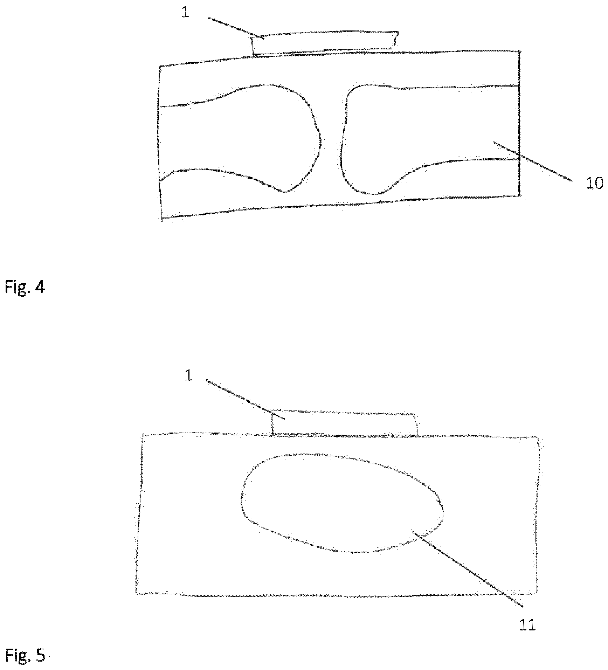

In Fig. 4 ist ein Querschnitt durch eine beliebige Therapieanordnung, zur Therapie eines Gelenks bzw. Knochens 10 dargestellt. Der Therapiekörper 1 wird dabei auf bestimmte, für die Behandlung des Gelenks bzw. Knochens 10 relevante Akupunkturpunkte, Meridiansysteme, Triggerpunkte, Reflexzonen oder sonstige Punkte auf die Haut des Patienten (menschliches oder tierisches Lebewesen) aufgelegt.

Fig. 5 zeigt einen Querschnitt durch eine beliebige Therapieanordnung, zur Therapie eines Organs, einer Zelle oder sonstiger Bestandteile 11 des menschlichen oder tierischen Körpers sowie einer Pflanze. Der Therapiekörper 1 wird dabei auf bestimmte, für die Behandlung des Organs, einer Zelle oder sonstiger Bestandteile 11 relevante Akupunkturpunkte, Meridiansysteme, Triggerpunkte, Reflexzonen oder sonstige Punkte auf die Haut des Patienten (menschliches oder tierisches Lebewesen) oder einer Pflanze aufgelegt.

Nach einer zweiten Ausführungsform der Erfindung besteht der Therapiekörper 1 aus keinem Polarisationsfilter 4, keinem, einem oder einer beliebigen Anzahl an Farbfiltern 5 und einem Spiegel 6. Der Spiegel kann sowohl als Oberflächenspiegels 6a als auch als Unterflächenspiegel 6b ausgebildet sein. Die therapeutische Wirkung ist für beide Ausführungen des Spiegels 6a und 6b identisch. Durch Weglassen des Polarisationsfilters 4 werden die Lichtstrahlen vor dem Farbfilter 5 nicht zusätzlich polarisiert. Die therapeutische Wirkung wird dadurch nicht beeinträchtigt.

Der Therapiekörper 1 wird vorzugsweise durch nicht dargestellte klebetechnische Haftmittel an den jeweiligen therapeutischen Auflagestellen, das heißt Akupunkturpunkten, Meridiansystemen, Triggerpunkten, Reflexzonen oder sonstigen Punkten auf die Haut des Patienten (menschliches oder tierisches Lebewesen) oder einer Pflanze aufgebracht. Eine Anordnung der Therapiekörper erfolgt je nach Behandlungsschwerpunkt in zueinander regelmäßigen und/oder unregelmäßigen Abständen.

Vorzugsweise werden die Therapiekörper 1 in einer Therapiezeit von 30 min bis zu 24 Stunden angewandt. Die Therapiekörper 1 sind täglich anwendbar und können in regelmäßigen oder unregelmäßigen Zeitabständen über einen kürzeren und/oder längeren und/oder individuellen Therapiezeitraum von einigen Stunden bis zu mehreren Monaten angewandt werden.

Die Lichtwellen-Therapievorrichtung kann vorteilhaft bei allen Erkrankungen eingesetzt werden, vorzugsweise bei chronischen und/oder wiederkehrenden Krankheitssymptomen. Als Vorteilhaft kann auch der Wegfall von Medikamenteneinnahmen, sowie die schonende Therapiemethode hervorgehoben werden. Durch den direkten Einfluss auf die menschlichen und/oder tierischen und/oder pflanzlichen Zellen kann nach Abschluss der Therapie der Patient als vollständig "gesund" gelten.

### Bezugszeichenliste:

- 1: Therapiekörper
- 2: obere Außenhülle
- 3: untere Außenhülle
- 4: Polarisationsfilter
- 5: Farbfilter
- 6: Spiegel
- 6a: Oberflächenspiegel
- 6b: Unterflächenspiegels
- 6c: Reflexfolie
- 7: Ronden
- 8: Label
- 9: Muskels
- 10: Knochens
- 11: Bestandteile

## Patentansprüche

1. Lichtwellen-Therapievorrichtung, welche durch einen auf die Haut eines menschlichen oder tierischen Lebewesens sowie auf die Oberfläche eines pflanzlichen Wesens aufbringbaren Therapiekörper (1) ausgebildet ist, aufweisend: eine obere Außenhülle (2), die relativ in Axialrichtung zu einer unteren Außenhülle (3) angeordnet ist, wobei innerhalb der Außenhülle unterschiedliche Arten und eine variable Anzahl von Polarisationsfiltern (4), Farbfiltern (5), Spiegelflächen (6), Ronden (7) und Label (8) derart angeordnet sind, dass der Therapiekörper (1) mit den unter der Haut angeordneten Zellen (11) und/oder Organen (11) und/oder Muskeln (9) und/oder Knochen und/oder Gelenken (10) und/oder andern Bestandteilen (11) des menschlichen sowie tierischen Körpers oder der Pflanze in Wirkverbindung tritt.

2. Lichtwellen-Therapievorrichtung nach Anspruch 1, wobei die obere Außenhülle (2) durch eine klebtechnische Verbindung mittels Kleber -vornehmlich PMMA- mit der unteren Außenhülle (3) verbunden ist, wobei eine Verbindung der oberen Außenhülle (2) mit der unteren Außenhülle (3) durch jede technisch bekannte Verbindungsart hergestellt werden kann.

3. Lichtwellen-Therapievorrichtung nach Anspruch 2, wobei die obere Außenhülle (2) und die untere Außenhülle (3) aus zertifizierten, biokompatiblen PMMA (z.B. Altuglas®SG7) ist.

4. Lichtwellen-Therapievorrichtung nach wenigstens einem der Ansprüche 1 bis 3, wobei die obere Außenhülle (2) ein Abmaß von 20 bis zu 150 mm und die untere Außenhülle (3) ein Abmaß von 20 bis zu 150 mm aufweist, wobei die Dicke des Therapiekörpers (1) je nach Spiegelfläche variiert und wobei der Durchmesser des Therapiekörpers (1) je nach Größe der Außenhülle (3) variieren kann.

5. Lichtwellen-Therapievorrichtung nach wenigstens einem der Ansprüche 1 bis 4, wobei der Therapiekörper (1) keinen oder einen Polarisationsfilter (4) aufweist und wobei der Polarisationsfilter (4) zwischen oberer Außenhülle (2) und Farbfilter (5) angeordnet ist, wobei der Polarisationsfilter (4) auch beliebig im Therapiekörper (1) platziert sein kann.

6. Lichtwellen-Therapievorrichtung nach wenigstens einem der Ansprüche 1 bis 5, wobei der Therapiekörper (1) keinen, einen oder eine unbestimmte Anzahl an Farbfiltern (5) aufweist und wobei der Farbfilter (5) zwischen Polarisationsfilter (4) und Spiegel (6) angeordnet ist, wobei der Farbfilter (5) so angeordnet ist, dass 2 Wellenlängen gleichzeitig reflektiert werden können.

7. Lichtwellen-Therapievorrichtung nach Anspruch 1 oder 5, wobei der Polarisationsfilter (4) aus folgendem Material: PMMA und/oder Glas und/oder Folie und/ oder einem anderen Material ist und das Licht von 200 nm bis 1400 nm polarisiert.

8. Lichtwellen-Therapievorrichtung nach wenigstens einem der Ansprüche 1 bis 7, wobei der Farbfilter (5) aus folgendem Material: PMMA und/oder Glas und/ oder Polyester und/oder einem anderen Material ist.

9. Lichtwellen-Therapievorrichtung nach wenigstens einem der Ansprüche 1 bis 8, wobei der Farbfilter (5) in folgender Farbgebung ausgebildet sein kann - lila, blau, hellblau, grün, gelb, orange, rot, braun, schwarz, weiß -, wobei der Farbfilter (5) je nach Farbgebung unterschiedliche Wellenlängen des Lichtes filtert.

10. Lichtwellen-Therapievorrichtung nach wenigstens einem der Ansprüche 1 bis 9, wobei die unterschiedlichen Farbfilter (5) beliebig miteinander kombiniert werden können.

11. Lichtwellen-Therapievorrichtung nach Anspruch 1, wobei die Therapievorrichtung (1) einen Spiegel (6) oder Reflexfolie (6c) aufweist und wobei der Spiegel (6) zwischen Farbfilter (5) und Ronde (7) angeordnet ist, wobei der Spiegel (6) oder die Reflexfolie (6c) auch beliebig im Therapiekörper (1) platziert sein kann.

12. Lichtwellen-Therapievorrichtung nach wenigstens einem der Ansprüche 1 bis 11, wobei die Therapievorrichtung (1) einen Spiegel (6) aufweist, wobei der Spiegel (6) je nach Ausführungsform, als Oberflächenspiegel (6a) oder als Unterflächenspiegel (6b) oder als Reflexfolie (6c) ausgebildet ist.

13. Lichtwellen-Therapievorrichtung nach wenigstens einem der Ansprüche 1 bis 12, wobei der Spiegel (6) einen Durchmesser von bis zu 40mm besitzt und wobei der Oberflächenspiegel (6a) eine Dicke von bis zu 1mm und der Unterflächenspiegel (6b) eine Dicke von bis zu 2mm aufweist.

14. Lichtwellen-Therapievorrichtung nach wenigstens einem der Ansprüche 1 bis 13, wobei die Oberfläche des Oberflächenspiegels (6a) mit einer Aluminium-Quarzschicht (Al+SiO₂) beschichtet ist, wobei der Oberflächenspiegel (6a) eine Wellenlänge von 200 nm bis zu 1400 nm reflektiert.

15. Lichtwellen-Therapievorrichtung nach wenigstens einem der Ansprüche 1 bis 14, wobei die Unterfläche des Unterflächenspiegels (6b) mit Aluminium beschichtet ist, wobei der Unterflächenspiegel (6b) eine Wellenlänge von 200 nm bis zu 5µm reflektiert.

16. Lichtwellen-Therapievorrichtung nach wenigstens einem der Ansprüche 1 bis 15, wobei der Therapiekörper (1) eine bis zehn Ronden (7) aufweist, wobei die Ronde als Füllmaterial bzw. Platzhalter für die Bereiche zwischen Spiegel (6, 6a, 6b) oder Reflexfolie (6c) und Label (8) dient, wobei die Ronden (7) auch beliebig im Therapiekörper (1) platziert sein können.

17. Lichtwellen-Therapievorrichtung nach wenigstens einem der Ansprüche 1 bis 16, wobei eine Ronde (7) einen Durchmesser von bis zu 40mm besitzt und wobei eine Ronde (7) eine Dicke von bis zu 0,5 mm aufweist und wobei eine die Ronde (7) aus Aluminium ausgebildet ist.

18. Lichtwellen-Therapievorrichtung nach wenigstens einem der Ansprüche 1 bis 17, wobei das Label (8) zur äußeren Kenntlichmachung des Therapiekörpers (1) dient und wobei das Label (8) im Bereich zwischen Ronde (7) und unterer Außenhülle (3) angeordnet ist, wobei das Label (8) auch beliebig im Therapiekörper (1) platziert sein kann.
